(19) Europäisches Patentamt
European Patent Office
Office européen des brevets

(11) **EP 4 386 367 B1**

(12) **EUROPEAN PATENT SPECIFICATION**

(45) Date of publication and mention
of the grant of the patent:
**06.11.2024 Bulletin 2024/45**

(21) Application number: **23215775.0**

(22) Date of filing: **12.12.2023**

(51) International Patent Classification (IPC):
**G01N 27/02** $^{(2006.01)}$ **G01N 33/38** $^{(2006.01)}$

(52) Cooperative Patent Classification (CPC):
**G01N 27/023; G01N 33/383;** G01N 27/20;
G01N 27/24

(54) **APPARATUS FOR DIAGNOSTICS OF DISPERSED REINFORCEMENT IN CEMENTITIOUS COMPOSITE**

VORRICHTUNG ZUR DIAGNOSE VON DISPERGIERTER VERSTÄRKUNG IN ZEMENTVERBUNDSTOFF

APPAREIL POUR LE DIAGNOSTIC DE RENFORCEMENT DISPERSE DANS UN COMPOSITE CIMENTAIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **12.12.2022 CZ 20220521**

(43) Date of publication of application:
**19.06.2024 Bulletin 2024/25**

(73) Proprietor: **Ceske vysoke uceni technicke v Praze
160 00 Praha 6 - Dejvice (CZ)**

(72) Inventors:
• **PAPEZ, Vaclav
10000 Praha 10 (CZ)**
• **KUNZEL, Karel
28802 Nymburk (CZ)**

(74) Representative: **Kratochvil, Vaclav
Patent and Trademark Office
P.O. Box 26
295 01 Mnichovo Hradiste (CZ)**

(56) References cited:
**US-A- 5 119 022 US-A- 5 528 142**

• **WU DEHUI ET AL: "Self-resonance eddy-current testing method in noncontact detection of carbon fiber reinforced plastics", COMPOSITE STRUCTURES, ELSEVIER SCIENCE LTD, GB, vol. 259, 7 November 2020 (2020-11-07), XP086448079, ISSN: 0263-8223, [retrieved on 20201107], DOI: 10.1016/J.COMPSTRUCT.2020.113247**

EP 4 386 367 B1

**Description**

Technical Field

A structure enabling non-destructive evaluation of the concentration and degree of orientation of ferromagnetic fibres, which forms the dispersed reinforcement in the cement composite, is solved.

Background Art

The orientation of fibres in cementitious composite significantly affects its final mechanical properties. Beams with more fibres in the tensile stress direction have higher tensile strength and can support higher loads, see publication S.-T. Kang, J.-K. Kim, The relation between fiber orientation and tensile behavior in an Ultra High Performance fiber Reinforced Cementitious Composites (UHPFRCC), Cem. Concr. Res. 41 (10) (2011) 1001-1014, https://doi.org/10.1016/j.cemconres.2011.05.009. The degree of fibre orientation in cementitious composites is critical for structural diagnostics. The orientation of ferromagnetic fibres can be evaluated according to the magnetic properties of the composite, and non-destructive testing of fibre orientation can be performed by magnetic field.

Diagnosis of the composite with ferromagnetic fibres is performed using the measuring coil into which the measured sample is inserted. Changes in the parameters of the measuring coil that are caused by insertion of the diagnosed body into the measuring coil are evaluated. The inductance of the measuring coil is relatively little affected by the ferromagnetic fibres in the composite. The change in inductance is around 1 to 2% and corresponds to the maximum difference in the volume of ferromagnetic material inserted into the measuring coil.

This issue is dealt with in publications by L. Ferrara, M. Faifer, M. Muhaxheri, S. Toscani - A magnetic method for non destructive monitoring of fiber dispersion and orientation in steel fiber reinforced cementitious composites. Part 2: correlation to tensile fracture toughness, Mater. Struct. 45 (4) (2012) 591-598 and by L. Ferrara, M. Faifer, S. Toscani - A magnetic method for non destructive monitoring of fiber dispersion and orientation in steel fiber reinforced cementitious composites - part 1: method calibration, Mater. Struct. 45 (4) (Apr. 2012) 575-589, https://doi.org/10.1617/s11527-011-9793-y. Also "Self-resonance eddy-current testing method in noncontact detection of carbon fiber reinforced plastics" by Dehui et al. Composite Structures 259 (2021) 113247, https://doi.org/10.1016/j.compstruct.2020.113247 discloses Eddy current testing for carbon fiber reinforced plastics.

The quality factor of the measuring coil shows greater sensitivity to the composition of the composite. The power dissipation resulting from hysteresis losses and eddy current induction in ferromagnetic fibres significantly increase the total losses in the measuring coil and cause a decrease in its quality factor.

$$Q = \frac{S}{P_R + P_I},$$

where

$S$    is the reactive power in the coil
$P_R$   is the active power dissipation in the coil conductor
$P_I$   is the coil power dissipation resulting from induced currents in ferromagnetic fibres.

Ferromagnetic fibres can be represented as a closed circuit consisting of their equivalent resistance and equivalent inductance, which is coupled by mutual inductance to the measuring coil. Since the magnitude of mutual inductance is decisively influenced by the angle of the fibre with respect to the axis of the measuring coil, the power dissipation caused in the coil by the influence of ferromagnetic fibres and ultimately also the quality factor of the measuring coil depend on this angle.

Due to the frequency dependence of the electrical and magnetic properties of the fibres, the power dissipation is also frequency dependent. At the frequency at which the impedance of the replacement fibre circuit is adapted to the impedance of the measuring coil, the damping of the coil is maximal and the decrease of its quality factor is maximal and can reach up to 50%, see article K. Künzel et al., 'Electromagnetic Properties of Steel Fibres for Use in Cementitious Composites, Fibre Detection and Non-Destructive Testing', Materials, vol. 14, no. 9, Art. No. 9, Jan. 2021, doi: 10.3390/ma14092131.

A measuring coil with an inductance of several tens of $\mu$H, a measuring frequency of several MHz and an indication of the quality factor of the measuring coil in the range of 50 to 500 is optimal for measuring the fibre concentration in the composite and its orientation.

Targeted fibre orientation in the concrete precast increases the strength of the precast in the fibre orientation direction while maintaining the required fibre concentration. Measurement of the quality factor thus allows to control the success of fibre orientation in the concrete precast, preferably by comparative measurement of the sample before and after exposure to the magnetic field. Such control is very important with respect to the sensitivity of the successful fibre orientation by the magnetic field to the proper preparation of the concrete mixture.

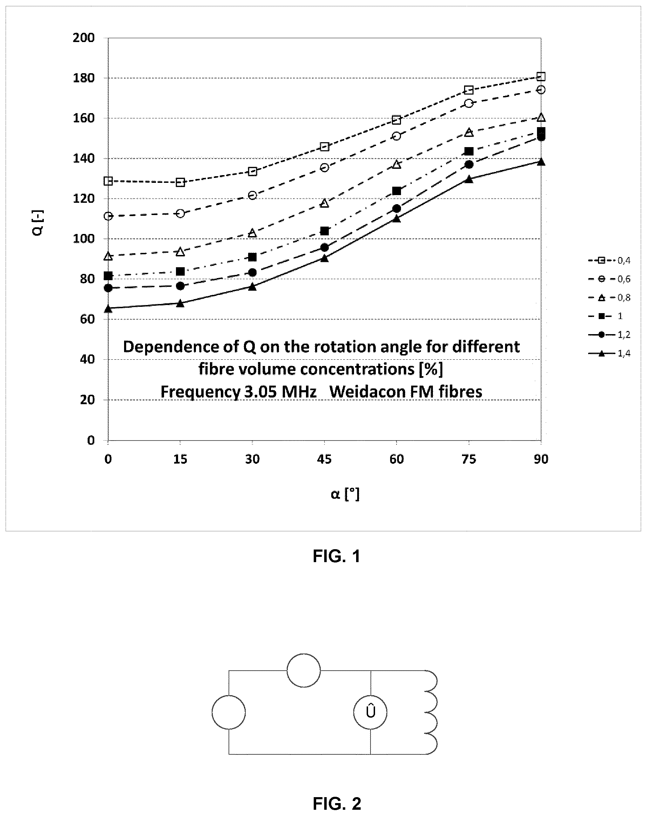

Chart in Fig. 1 shows the results of measuring the quality factor of coil Q into which cylindrical samples with oriented Weidacon FM fibres with different volume concentrations were inserted. The samples were angled relative to the axis of the measuring coil so that the angle $\alpha$=0° indicates the orientation of the fibres in the direction of the coil axis, $\alpha$=90° perpendicular to the coil axis.

Individual curves document the influence of fibre orientation on the measured quality factor of the measuring coil. The curves differ in the fibre volume concentration in the sample. The quality factor increases with decreasing concentration, which documents the upward shift of the measured curves. Individual curves show the increase of the detected quality factor increasing with the increasing rotation angle relative to the axis of the measuring coil.

The most commonly used methodology for determining the coil quality factor in the above frequency range is a comparison of the sizes of the real and imaginary components of its complex immittance, which was determined according to the generalized Ohm's law for phasors according to the voltage phasor at the coil terminals and the current phasor flowing through the coil. The usual design of an impedance meter operating on this principle is shown in Fig. 2. The measuring signal generated by the measuring signal generator $\underline{G}$ is fed to the terminals of the measured coil $\underline{L}$ or any single gate, wherein the flowing current and voltage at the measuring terminals are monitored by a vector voltmeter $\hat{\underline{U}}$ and an ammeter $\hat{\underline{I}}$ , the impedance is determined according to the phasor ratio. The quality factor can be easily determined according to the phase deviation $\delta$ of the voltage and current phasors, independent of the phasor size and independent of the measuring frequency.

$$Q = \frac{1}{tg\ \delta}$$

The method can be modified by adjusting or stabilizing the amount of current flowing through the measuring circuit to a constant value, thereby becoming an impedance directly proportional to the voltmeter indicated voltage phasor. When measuring the fibre concentration in the composite and its orientation, the biggest problem of this methodology is represented by the measurement of the relatively high quality factor of the measuring coil. Changes in the quality factor that are sought are at the level of measurement inaccuracy.

The accuracy of the quality factor measurement can be determined, when measuring with impedance meters, by the more frequently reported accuracy of the phase angle or loss factor measurement. The manufacturer's guaranteed measurement accuracy of the highest quality meters is a few tenths of a degree for the phase angle, or a few thousandths for the loss factor. This corresponds to a measurement accuracy for the quality factor of approximately 10% for $Q{\approx}100$ values, which deteriorates up to 30% for $Q{\approx}500$ values, which does not allow reliable determination of changes in the quality factor of the measuring coil due to ferromagnetic fibres.

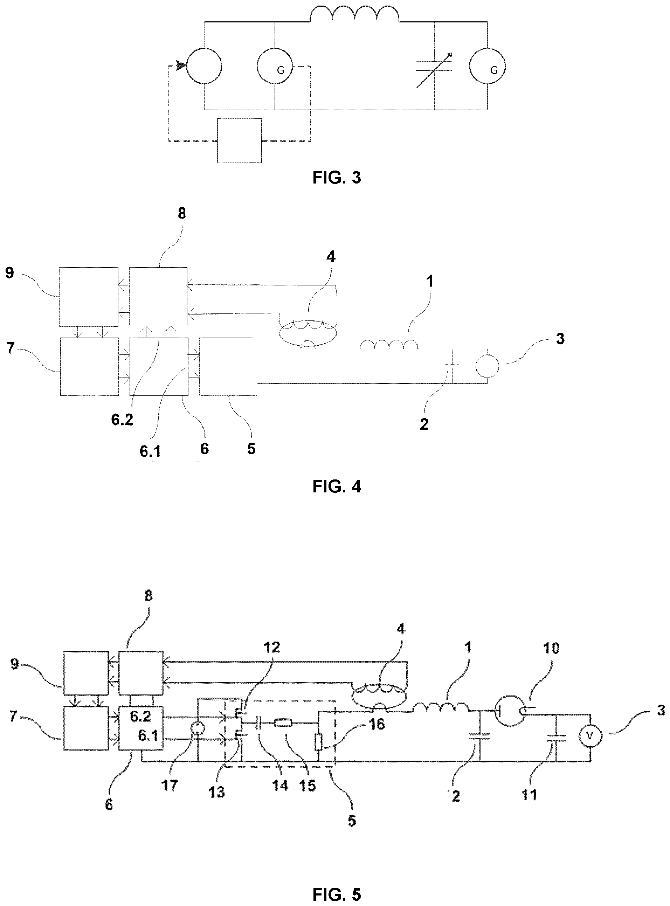

Another methodology used to determine the coil quality factor in the above frequency range is the measurement of voltage conditions in the series resonant circuit in which the coil is included. The principle connection of an apparatus operating this methodology, which is usually called a quality factor meter or Q-meter, is shown in Fig. 3.

The measuring circuit consists of a series connection of the measured coil $\underline{L}$, the variable capacitor $\underline{C}$ and the measuring signal generator $\underline{G}$. The capacitance of the variable capacitor $\underline{C}$ is adjusted during measurement so that a resonance occurs at the measuring frequency $f_m$ in the series resonant circuit it forms together with the coil $\underline{L}$.

$$f_m = \frac{1}{2\pi\sqrt{LC}}$$

Under this condition, in the series resonant circuit, the impedance is compensated by the reactions of the coil $\underline{L}$ and the capacitor $\underline{C}$, and if a sufficiently high-quality capacitor is used and its losses can be neglected, the current I of the circuit is determined exclusively by the output voltage $U_G$ of the generator $\underline{G}$ and the series damping resistance $\overline{R_s}$ of the coil $\underline{L}$.

$$I = \frac{U_G}{R_S}$$

The voltage Uc on the capacitor $\underline{C}$ is determined by the current I and the reactance of the capacitor $\underline{C}$ and is the same as the voltage on the reactance of the coil $\underline{L}$.

$$U_C = IX_C = IX_L$$

The coil quality factor Q is equal to the voltage ratio $U_C/U_G$, which is often referred to as the enhancement factor.

$$Q = \frac{X_L}{R_S} = \frac{U_C}{U_G}$$

If the voltage $U_G$ at the output of the measuring signal generator $\underline{G}$ is kept constant during measurement, the quality factor is directly proportional to the oscillated voltage Uc at the capacitor $\underline{C}$, and the voltmeter indicating this voltage tends to be directly calibrated in the values of the quality factor Q.

The constant voltage level $U_G$ at the output of the measuring signal generator $\underline{G}$ is adjusted by the operator of the instrument according to the voltmeter indicated voltage $U_G$, or a small resistance is serially included in the measuring circuit on which the voltage $\mathbf{U_G}$ is generated by the current flow, the magnitude of which is indicated by the thermoelectric ammeter and adjusted to the selected value by the operator. Such solution is, for example, given in document JPS57135348. For quality instruments, the constant level of the voltage $U_G$ is set by the controller REG, which according to the sample of the voltage $U_G$ controls the output power of the measuring signal generator G so that its output voltage is constant and independent of the load impedance, or the measuring signal generator G is designed to have a minimum and negligible internal impedance at the output.

When measuring with quality factor meters, the achievable measurement accuracy is better. For a quality factor in the range of 100 to 300, a measurement accuracy of 3% to 4% can usually be achieved; for a quality factor in the range of 300 to 500, a measurement accuracy of 6% to 7% can usually be achieved. This measurement accuracy is sufficient to determine changes in the quality factor of the measuring coil due to ferromagnetic fibres.

However, the use of a measuring method to determine changes in the quality factor of the measuring coil due to ferromagnetic fibres complicates the need to adjust the measuring apparatus, which must ensure a resonant condition when measuring by changing the resonant capacitance or measuring frequency. The need for continuous adjustment is the most serious shortcoming of this measuring procedure, which virtually excludes its use for continuous or automated measurements.

Summary of the Invention

The above disadvantages are eliminated by the apparatus for measuring the concentration of ferromagnetic fibres in the composite according to the present proposal. Its essence is that it consists of a resonant circuit consisting of a capacitor and a measuring coil, which, by changing the operating frequency, is kept in resonance and the quality factor of which is determined according to the enhancement factor.

An apparatus for diagnosing dispersed reinforcement in cementitious composites includes a measuring signal generator and an LC resonant circuit, where the measuring coil is connected using one terminal to a terminal of a resonant capacitor to which an indication high frequency voltmeter is connected in parallel. The second terminal of the resonant capacitor is connected to the ground bus. The essence of the new solution is that the resonant capacitor, unlike the previously known solutions, is a fixed capacitor and the second terminal of the measuring coil is connected to the first pin of the primary winding of the current measuring transformer, which is connected by the second pin to the second terminal of the output gate of the power amplifier, the first terminal of which is connected to the ground bus. The secondary winding of the current measuring transformer is connected to the first input gate of the phase detector, the second input gate of which is connected to the comparison output of the measuring signal generator formed by the second output terminals. The output gate of the phase detector is connected to an integrator input, the output of which is connected to a voltage controlled oscillator control input having an output connected to the input terminals of a measuring signal generator, the first output terminals of which are connected to the input terminals of a power amplifier.

In one possible embodiment, the indication voltmeter is a DC voltmeter, which is bridged by an integration capacitor connected by one end to the ground bus and the other end to the vacuum diode cathode. The vacuum diode anode is connected to a common point of the coil and the resonant capacitor.

Another possible implementation is when the power amplifier at its input consists of two serially connected MOS switches forming a power pulse source of the measuring signal. A DC power supply is connected to the MOS switches. An isolation capacitor is then connected to the interconnection node of these MOS switches by one pin, which is connected by another pin to one end of the first resistor of the resistor divider, the other end of which is connected to one end of the second resistor. The other end of the second resistor is connected to the ground bus. The common joint of the first and second resistors is the output of the power amplifier connected via the primary winding of the current measuring transformer to the measuring coil.

The advantage of the new measuring apparatus solution is the automatic validity of the resonant condition during measurements enabled by a special measuring circuit. The resonance in the measuring circuit is not adjusted as in known quality factor meters by changing the capacitance of the variable capacitor, but at a constant capacitance of the capacitor by changing the measuring frequency. The error resulting from the determination of the quality factor by this procedure can be compensated according to the change in the measuring frequency, or it can be neglected because it reaches a maximum of several tenths of one percent of the measured value.

The control of the measuring frequency in the apparatus is provided by a feedback control loop of the phase lock, which compares the phase of the excitation voltage of the LC resonant circuit with the phase of the current flowing through it and adjusts the measuring frequency using a voltage controlled oscillator so that the instantaneous deviation of the phases is minimal. This automatically ensures continuous observance of the resonance condition in the LC circuit during measurement, allowing evaluation of the quality factor of the LC resonance circuit directly according to the amount of voltage oscillated on the resonance capacitor.

For previously known apparatuses, the quality factor of the measuring coil is monitored using a complex immittance meter, which degrades the measurement by its high measurement uncertainty, or a resonant meter of the quality factor, with which the measurement, for each value of the quality factor read, must be individually adjusted.

## Brief Description of the Drawings

The apparatus for diagnosing dispersed reinforcement in cementitious composite will be further clarified using the attached drawings. Fig. 1 shows the course of dependence of the quality factor Q of the coil with oriented ferromagnetic fibres of different volume concentration and with different angular deviation of orientation direction from the coil axis. Fig. 2 and Fig. 3 illustrate the state of the art. The basic wiring according to the new solution is shown in Fig. 4 and its further modification is shown in Fig. 5.

## Description of the Embodiments of the Invention

The principle arrangement of the apparatus for diagnostics of dispersed reinforcement in cementitious composite is shown in Fig. 4.

The measuring coil 1 is connected by one terminal to the resonant capacitor 2, to which a high frequency voltmeter 3 indicating the oscillating voltage is connected by the second terminal to the ground bus. The second pin of the measuring coil 1 is connected to the first pin of the primary winding of the current measuring transformer 4, which is connected by the second pin to the second terminal of the output gate of the power amplifier 5. The first terminal of the output gate of the power amplifier 5 is connected to the ground bus. The secondary winding of the current measuring transformer 1 is connected to the first input gate of the phase detector 8, the second input gate of which is connected to the comparison output of the measuring signal excitation generator 6 at the second output terminals 6.2, which is a sample of the output voltage of the measuring signal excitation generator 6 and which is fed to the input of the power amplifier 5. The output gate of the phase detector 8 is connected to the input of the integrator 9, the output of which is connected to the control input of the voltage controlled oscillator 7, which by its high frequency output signal controls the frequency of the output and comparison signal of the measuring signal excitation generator Q. The first output terminals 6.1 of the measuring signal excitation generator 6 are connected to the input terminals of the power amplifier 5.

The feedback loop consisting of the measuring signal excitation generator 6, the power amplifier 5, the LC resonant circuit, the current measuring transformer 4, the phase detector 8 and the integrator 9 forms a phase lock control system that compares the phase of the excitation voltage of the resonant circuit LC with the phase of the current flowing through it and adjusts the frequency of the voltage controlled oscillator 7 so that the instantaneous deviation of the phases is minimal. This automatically ensures continuous observance of the resonance condition in the LC circuit during the measurement allowing, at its constant excitation voltage, to evaluate the quality factor of the resonance circuit LC directly according to the magnitude of the voltage oscillated on the resonance capacitor 2, which is determined by the indication radio frequency voltmeter 3. Under the condition of minimizing resonance losses in the resonant capacitor 2 and its minimum damping by the connected indication high frequency voltmeter 3, the quality factor of the resonant circuit LC corresponds to the sought quality factor of the measuring coil 1.

The quality factor of the measuring coil 1 is measured at resonance in a resonant circuit consisting of the measuring coil 1 and a series-connected resonant capacitor 2, which is excited by a constant-level measuring signal from the power amplifier 5 and the oscillating voltage of which at the resonant capacitor 2 is indicated by a high frequency voltmeter 3.

The source of the measuring signal consists of a voltage controlled oscillator 7, the oscillation frequency of which is controlled by a phase lock loop, a measuring signal excitation generator 6 and a power amplifier 5. The output terminals of the voltage controlled oscillator 7 are connected to the input terminals of the measuring signal excitation generator 6, where a control signal for the power amplifier 5 is generated by a synchronous counter and decoders, which is fed to the first output terminals 6.1, and a comparison signal for the phase detector 8, which is fed to the second output terminals 6.2. The first output terminals 6.1 of the measuring signal excitation generator 6 are connected to the input terminals of the power amplifier 5, from the output of which the excitation signal for the measurement circuit is drawn.

The feedback branch of the phase lock loop consists of a current measuring transformer 4, a phase detector 8 and an integrator 9. The output signal from the secondary winding of the current measuring transformer 4, corresponding to the current sample in the measuring circuit, is fed to its output terminals, which are connected to the terminals of the first input gate of the phase detector 8. The terminals of the second input gate of the phase detector 8 are connected to the second output terminals 6.2 of the measuring signal excitation generator 6, from which the comparison signal for the phase detector 8 is drawn. The output terminals of the phase detector 8 are connected to the input terminals of the integrator 9, which forms the low frequency filter of the phase lock loop. From the output terminals of the integrator 9, which are connected to the input terminals of the voltage controlled oscillator 7, a control signal is supplied to the control input of the voltage controlled oscillator 7.

The essence of the new solution is therefore the construction of the measuring signal generator with the pulse waveform of the output signal and with the control of the frequency of the output signal by the phase lock circuit according to the instantaneous resonant frequency of the measuring resonant circuit.

The use of the impulse waveform of the excitation signal is made possible by the high selectivity of the measuring circuit because the series resonant circuit in this connection acts as a highly selective bandpass, its response to the impulse excitation signal is determined practically only by the 1st harmonic component of the spectrum of this signal, which corresponds to its resonant frequency.

The quality factor of the measuring resonant circuit is, as in sinusoidal excitation signal measurements, directly proportional to the oscillated voltage at the resonant capacitor 2 and can be determined by the ratio of the oscillated voltage to the output voltage of the measuring signal generator, which is corrected according to the coefficient of the 1st harmonic component of the spectrum of the pulse output signal of the measuring signal generator.

According to the determined quality factor, the success of the orientation of the fibres in the precast concrete and their concentration can be controlled, preferably by a comparative measurement of the sample before exposure in the magnetic field through which the fibres are to be oriented and after exposure. Such control is very important with respect to the sensitivity of the successful fibre orientation by the magnetic field to the proper preparation of the concrete mixture.

One example of the implementation of the apparatus for diagnostics of dispersed reinforcement in cementitious composite is shown in Fig. 5. In this example, the connection is analogous to Fig. 4, the circuit of the indication high frequency voltmeter 3 and the realization of the power amplifier 5 are improved.

A series resonant circuit consisting of a measuring coil 1 and a resonant capacitor 2, supplied with a measuring signal from a power amplifier 5, is used as the measuring circuit of the apparatus for diagnostics of dispersed reinforcement in cementitious composite. The oscillating voltage on the resonant capacitor 2 is evaluated by a peak voltmeter. To achieve the minimum damping of the resonant circuit with an indication voltmeter, the voltmeter is implemented as a high-end detector with a vacuum diode 10, with an integration capacitor 11 and a special DC high frequency voltmeter 3 with a measuring range of approximately 100 V and an input resistance of at least 100 MΩ.

A power amplifier 5 consists here at its input of two serially connected MOS switches 12, 13 forming a power pulse source of the measuring signal. A DC power supply 17 is connected to MOS switches 12 and 13. An isolation capacitor 14 is connected to the interconnection node of these MOS switches 12, 13 by one pin, which is connected by a second pin to one end of the first resistor 15 of the resistance divider. Its other end is connected to one end of the second resistor 16 of the resistor divider having the other end connected to the ground bus. The common joint of the first resistor 15 and the second resistor 16 is the output of the power amplifier 5 connected via the primary winding of the current measuring transformer 1 to the measuring coil 1.

The input signals from the measuring signal excitation generator 6 are controlled by two interconnected MOS switches 12 and 13, which together with the connected DC power supply 17 form the power pulse source of the measuring signal. Furthermore, at the node with the interconnection of MOS switches 12 and 13, an isolation capacitor 14 is connected by the first pin, which is connected by the second pin to the bleeder with resistors 15 and 16. The resistance value of the second resistor 16 is selected small so that the output voltage of the divider resistors 15 and 16 is not significantly influenced by the resonant circuit measuring coil 1 and resonant capacitor 2. The resistance value of the first resistor 15 is selected so that the MOS switches 12 and 13 are not overloaded with the load current, while the signal level of approximately 0.2 V, which is sufficient for measurement with the required accuracy, is present at the output of the divider with resistors 15 and 16.

Industrial Applicability

The solution is applicable wherever the level of orientation of ferromagnetic fibres in a cement mixture with dispersed reinforcement consisting of ferromagnetic fibres is evaluated. Targeted fibre orientation is used to achieve anisotropic mechanical properties of precast parts in order to increase strength in the desired directions. The proposed solution allows checking the success of such orientation.

**Reference Signs List**

| | |
|---|---|
| 1 | measuring coil |
| 2 | resonant capacitor |
| 3 | high frequency voltmeter |
| 4 | current measuring transformer |
| 5 | power amplifier |
| 6 | measuring signal excitation generator |
| | 6.1 first output terminals |
| | 6.2 second output terminals |
| 7 | voltage controlled oscillator |
| 8 | phase detector |
| 9 | integrator |
| 10 | vacuum diode |
| 11 | integration capacitor |
| 12 | MOS switch |
| 13 | MOS switch |
| 14 | first resistor |
| 15 | second resistor |
| 16 | power supply |

**Claims**

1. An apparatus for diagnosing dispersed reinforcement in a cementitious composite comprising a measuring signal excitation generator (6) and an LC resonant circuit, where a measuring coil (1) is one terminal connected to one terminal of a resonant capacitor (2) to which an indication high frequency voltmeter (3) is connected in parallel and a second terminal of which is connected to a ground bus **characterized in that**

- the resonant capacitor (2) is a fixed capacitor and
- the second terminal of the measuring coil (1) is connected to a first pin of a primary winding of a current measuring transformer (4),
- a second pin of the primary winding of the current measuring transformer (4) is connected to a second terminal of an output gate of a power amplifier (5),
- a first terminal of the output gate is connected to the ground bus and
- a secondary winding of the current measuring transformer (4) is connected to a first input gate of a phase detector (8),
- a second input gate of the phase detector (8) is connected to a comparison output of the measuring signal excitation generator (6), wherein
- the measuring signal excitation generator (6) is formed by second output terminals (6.2), and
- an output gate of the phase detector (8) is connected to an integrator (9) input,
- an integrator (9) output is interconnected with a control input of a voltage controlled oscillator (7),
- an output the voltage controlled oscillator (7) is connected to input terminals of the measuring signal excitation generator (6),
- first output terminals (6.1) of the measuring signal excitation generator (6) are connected to input terminals of the power amplifier (5).

2. The apparatus according to claim 1 **characterized in that** the indication high frequency voltmeter (3) is a DC voltmeter and is bridged by an integration capacitor (11) connected at one end to the ground bus and at the other end to a cathode of a vacuum diode (10), an anode of the vacuum diode (10) is connected to a common point of the measuring coil (1) and the resonant capacitor (2).

3. The apparatus according to claim 1 or 2 **characterized in that** the power amplifier (5) consists at its input of two MOS switches (12, 13) connected in series forming a power pulse source of the measuring signal to which a DC power supply (17) is connected, and that to an interconnection node of these MOS switches (12, 13), an isolation capacitor (14) is connected by one pin, which isolation capacitor is connected by the second pin to one end of the first resistor (15) of the bleeder, the other end of which is connected to one end of the second resistor (16) having the other end connected to the ground bus, and where the common joint of the first resistor (15) and the second resistor (16) is the output of the power amplifier (5) connected via the primary winding of the current measuring transformer (4) to the measuring coil (1).

**Patentansprüche**

1. Vorrichtung zur Diagnose von Faserbewehrung in Zementverbundwerkstoffen umfassend einen Erregungsgenerator (6) für ein Messsignal und einen LC-Schwingkreis, wobei die Messspule (1) über eine Klemme mit einer Klemme des Resonanzkondensators (2) verbunden ist, dem ein anzeigendes Hochfrequenz-Voltmeter (3) parallel geschaltet ist und dessen andere Klemme mit einer Erdungsschiene verbunden ist, **dadurch gekennzeichnet, dass**

- der Resonanzkondensator (2) ein Festkondensator ist und
- die zweite Klemme der Messspule (1) mit dem ersten Anschluss der Primärwicklung des Strommesswandlers (4) verbunden ist,
- der zweite Anschluss der Primärwicklung des Strommesswandlers (4) mit der zweiten Klemme des Ausgangsgates des Leistungsverstärkers (5) verbunden ist,
- die erste Klemme des Ausgangsgates mit der Erdungsschiene verbunden ist und
- die Sekundärwicklung des Strommesswandlers (4) mit dem ersten Eingangsgate des Phasendetektors (8) verbunden ist,
- das zweite Eingangsgate des Phasendetektors (8) mit dem Vergleichsausgang des Erregungsgenerators (6) des von den zweiten Ausgangsklemmen (6.2) gebildeten Messsignals verbunden ist und
- das Ausgangsgate des Phasendetektors (8) mit dem Eingang des Integrators (9) verbunden ist,
- der Ausgang des Integrators (9) mit dem Steuereingang des spannungsgesteuerten Oszillators (7) verbunden ist
- der Ausgang des spannungsgesteuerten Oszillators (7) mit den Eingangsklemmen des Erregungsgenerators (6) des Messsignals verbunden ist
- die ersten Ausgangsklemmen (6.1) des Erregungsgenerators (6) des Messsignals mit den Eingangsklemmen des Leistungsverstärkers (5) verbunden sind.

2. Vorrichtung nach Anspruch 1, **dadurch gekennzeichnet, dass** der anzeigende Hochfrequenz-Voltmeter (3) ein Gleichspannungsmesser ist und von einem integrierenden Kondensator (11) überbrückt wird, der an einem Ende mit der Erdungsschiene und am anderen Ende mit der Kathode der Vakuumdiode (10) verbunden ist, deren Anode mit einem gemeinsamen Punkt der Spule (1) und des Resonanzkondensators (2) verbunden ist.

3. Vorrichtung nach Anspruch 1 oder 2, **dadurch gekennzeichnet, dass** der Leistungsverstärker (5) an seinem Eingang zwei seriell geschaltete MOS-Schalter (12, 13) umfasst, um eine Leistungsimpulsquelle des Messsignals zu bilden, an die eine Gleichstromversorgung (17) angeschlossen ist, und dass ein Trennkondensator (14) über einen Anschluss mit dem Verbindungsknoten dieser MOS-Schalter (12, 13) verbunden ist und über einen zweiten Anschluss mit einem Ende eines ersten Widerstands (15) eines Widerstandsteilers verbunden ist, dessen anderes Ende mit einem Ende des zweiten Widerstands (16) verbunden ist, dessen anderes Ende mit einer Erdungsschiene verbunden ist, und wobei der gemeinsame Anschluss des ersten Widerstands (15) und des zweiten Widerstands (16) der Ausgang des Leistungsverstärkers (5) ist, der über die Primärwicklung eines Strommesstransformators (4) mit einer Messspule (1) verbunden ist.

## Revendications

1. Dispositif pour diagnostiquer les renforts dispersés dans un composite de ciment contenant un générateur d'excitation (6) d'un signal de mesure et un circuit résonant LC, où une bobine de mesure (1) est reliée au moyen d'une borne à une borne du condensateur de résonance (2) auquel un voltmètre d'indication haute fréquence (3) est connecté en parallèle et dont une deuxième borne est connectée au bus de masse **caractérisé en ce que**

   - le condensateur de résonance (2) est un condensateur fixe et
   - la deuxième borne de la bobine de mesure (1) est reliée à une première broche terminale de l'enroulement primaire du transformateur de mesure (4) de courant,
   - la deuxième broche terminale de l'enroulement primaire du transformateur de mesure (4) de courant est connectée à la deuxième borne de la porte de sortie de l'amplificateur de puissance (5),
   - la première borne de la porte de sortie est connectée au bus de masse et
   - l'enroulement secondaire du transformateur de mesure (4) de courant est relié à la première porte d'entrée du détecteur de phase (8),
   - la deuxième porte d'entrée du détecteur de phase (8) est reliée à la sortie de comparaison du générateur d'excitation (6) du signal de mesure formé par les deuxièmes bornes de sortie (6.2) et
   - la porte de sortie du détecteur de phase (8) est reliée à l'entrée de l'intégrateur (9), j
   - la sortie de l'intégrateur (9) est reliée à l'entrée de commande de l'oscillateur commandé en tension (7)
   - la sortie de l'oscillateur commandé en tension (7) est reliée aux bornes d'entrée du générateur d'excitation (6) de signal de mesure
   - les premières bornes de sortie (6.1) du générateur d'excitation (6) du signal de mesure sont reliées aux bornes d'entrée de l'amplificateur de puissance (5).

2. Dispositif selon la revendication 1 **caractérisé en ce que** le voltmètre d'indication haute fréquence (3) est un voltmètre à courant continu et est shunté par un condensateur d'intégration (11) relié par une extrémité au bus de masse et par l'autre extrémité à la cathode d'une diode à vide (10) dont l'anode est connectée au point commun de la bobine (1) et du condensateur de résonance (2).

3. Dispositif selon la revendication 1 ou 2 **caractérisé en ce qu'à** son entrée, l'amplificateur de puissance (5) est constitué de deux commutateurs MOS (12, 13) reliés en série formant une source d'impulsions de puissance du signal de mesure, auxquels une source d'alimentation continue (17) est connectée, et au noeud d'interconnexion de ces commutateurs MOS (12, 13), un condensateur de dérivation (14) est connecté au moyen d'une sortie , ce dernier est relié par l'autre sortie à une extrémité d'une première résistance (15) d'un diviseur de résistance, dont l'autre extrémité est reliée à une extrémité de la seconde résistance (16) dont l'autre extrémité est reliée au bus de masse et où la liaison commune de la première résistance (15) et de la seconde résistance (16) est la sortie de l'amplificateur de puissance (5) reliée via l'enroulement primaire du transformateur de mesure (4) de courant à la bobine de mesure (1).

**FIG. 1**

**FIG. 2**

**FIG. 3**

**FIG. 4**

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S57135348 B

### Non-patent literature cited in the description

- **S.-T. KANG ; J.-K. KIM.** The relation between fiber orientation and tensile behavior in an Ultra High Performance fiber Reinforced Cementitious Composites (UHPFRCC). *Cem. Concr. Res.,* 2011, vol. 41 (10), 1001-1014, https://doi.org/10.1016/j.cemconres.2011.05.009
- **L. FERRARA ; M. FAIFER ; M. MUHAXHERI ; S. TOSCANI.** A magnetic method for non destructive monitoring of fiber dispersion and orientation in steel fiber reinforced cementitious composites. Part 2: correlation to tensile fracture toughness. *Mater. Struct,* 2012, vol. 45 (4), 591-598
- **L. FERRARA ; M. FAIFER ; S. TOSCANI.** A magnetic method for non destructive monitoring of fiber dispersion and orientation in steel fiber reinforced cementitious composites - part 1: method calibration. *Mater. Struct.,* April 2012, vol. 45 (4), 575-589, https://doi.org/10.1617/s11527-011-9793-y
- **DEHUI et al.** Self-resonance eddy-current testing method in noncontact detection of carbon fiber reinforced plastics. *Composite Structures,* 2021, vol. 259, 113247, https://doi.org/10.1016/j.compstruct.2020.113247
- **K. KÜNZEL et al.** Electromagnetic Properties of Steel Fibres for Use in Cementitious Composites, Fibre Detection and Non-Destructive Testing. *Materials,* January 2021, vol. 14 (9